# FASCICULE DE BREVET EUROPEEN

(11) **EP 3 298 126 B1**
(45) Date de publication et mention de la délivrance du brevet: **08.01.2020**
(21) Numéro de dépôt: 16727771.4
(22) Date de dépôt: 18.05.2016
(51) Int. Cl.: C12N 1/12, A23J 3/20, A23L 17/60

(54) **PROCÉDÉ FERMENTAIRE DE DÉCOLORATION DE LA BIOMASSE DE CHLORELLA PROTOTHECOIDES**
FERMENTATIVES VERFAHREN ZUM BLEICHEN VON BIOMASSE VON CHLORELLA PROTOTHECOIDEN
FERMENTATIVE METHOD FOR BLEACHING BIOMASS OF CHLORELLA PROTOTHECOIDES

(30) Priorité: 19.05.2015 FR 1554442
(43) Date de publication de la demande: 28.03.2018
(73) Titulaire: Corbion Biotech, Inc., South San Francisco, CA 94080 (US)
(72) Inventeur: LE RUYET, Marie, 59000 Lille (FR); SEGUEILHA, Laurent, 59520 Marquette Lez Lille (FR); DELAROCHE, Sylvain, 62219 Longuenesse (FR)
(74) Mandataire: Cabinet Becker et Associés
(86) Numéro de dépôt international: PCT/FR2016/051163
(87) Numéro de publication internationale: WO 2016/185133

(56) Documents cités:
- WO-A1-2014/117163
- WO-A2-2014/154787
- SHIHIRA-ISHIKAWA I ET AL: "Nutritional control of cell pigmentation in Chlorella Protothecoides with special reference to the degeneration of chloroplast induced by glucose", PLANT AND CELL PHYSIOLOGY, OXFORD UNIVERSITY PRESS, UK, vol. 5, no. 2, 1 janvier 1964 (1964-01-01) , pages 227-240, XP008164642, ISSN: 0032-0781
- TAKAHIRA OGAWA ET AL: "Bioenergetic analysis of mixotrophic growth inChlorella vulgaris andScenedesmus acutus", BIOTECHNOLOGY AND BIOENGINEERING., vol. 23, no. 5, 1 mai 1981 (1981-05-01), pages 1121-1132, XP055260409, US ISSN: 0006-3592, DOI: 10.1002/bit.260230519

## Description

La présente invention se rapporte à un procédé fermentaire de décoloration de la biomasse de microalgues, plus particulièrement du genre *Chlorella,* plus particulièrement encore de l'espèce *Chlorella protothecoides.*

Les algues, macro et micro, ont une richesse spécifique en grande partie inexplorée. Leur exploitation à des fins alimentaire, chimique ou bioénergétique est encore très marginale. Elles recèlent cependant des composants de grande valeur.

Les microalgues sont en effet des sources de vitamines, lipides, protéines, sucres, pigments et antioxydants.

Les algues et microalgues intéressent ainsi le secteur industriel qui les utilise pour la fabrication de compléments alimentaires, d'aliments fonctionnels, de cosmétiques, de médicaments ou pour l'aquaculture.

L'utilisation des biomasses de microalgues (et principalement leurs protéines) comme aliments est de plus en plus considérée pour trouver des sources alternatives à la demande croissante en protéines animales au niveau mondial (comme rapporté par la FAO).

L'Union européenne, depuis des années, souffre par ailleurs d'un déficit structurel en protéines végétales qui s'élève, ces dernières années, à plus de 20 millions de tonnes d'équivalent soja, aujourd'hui importés d'Amérique du Sud.

La production de masse de certaines microalgues riches en protéines est alors envisagée comme une possibilité de combler ce « déficit en protéines ».

Des analyses exhaustives et des études nutritionnelles ont montré que ces protéines d'algues sont équivalentes aux protéines végétales conventionnelles, voire de meilleure qualité.

Toutefois, en raison des coûts élevés de production ainsi que des difficultés techniques pour intégrer le matériel issu de microalgues dans des préparations alimentaires organoleptiquement acceptables, la diffusion large des protéines de microalgues est encore à ses balbutiements.

En effet, si des poudres d'algues par exemple fabriquées avec des algues cultivées photosynthétiquement dans des étangs extérieurs ou par photobioréacteurs sont disponibles dans le commerce, elles ont une couleur vert foncée (liée à la chlorophylle) et un goût fort, désagréable.

Même formulées dans des produits alimentaires ou comme compléments nutritionnels, ces poudres d'algues communiquent toujours cette couleur verte visuellement peu attrayante au produit alimentaire ou au complément nutritionnel et ont un goût désagréable de poisson ou la saveur d'algues marines.

Il existe donc toujours un besoin non satisfait de disposer de compositions de biomasse de microalgues du genre *Chlorella* de qualité organoleptique convenable permettant l'utilisation de celles-ci dans des produits alimentaires plus nombreux et diversifiés.

Une première solution proposée a été de sélectionner des variants de Chlorelles présentant de faible teneur ou une absence de pigments chlorophylliens. C'est ainsi que *Prototheca* est classiquement décrite comme une Chlorelle incolore.

Une seconde solution a consisté à irradier aux rayons X ou UV, à traiter par des agents chimiques (NTG) ou physique (traitement thermique) une souche parentale afin de sélectionner des mutants dépigmentés.

La culture de ces mutants est réalisée préférentiellement en conditions hétérotrophiques (à l'obscurité et en présence d'une source nutritive carbonée, comme le glucose) afin de maintenir une pression de sélection.

Cependant, ces solutions techniques ne garantissent pas automatiquement la stabilité de ces variants dépigmentés, ni la préservation de la qualité, richesse et/ou diversité des autres composants d'intérêt de la biomasse.

Il est constaté (WO 2014/117163) une réduction de pigmentation d'une souche de Chlorella cultivé en hétérotrophie à un taux d'oxygène dissous de 60-70%, comparé à un taux d'oxygène dissous de 30-40%.

Il existe donc toujours un besoin non satisfait de disposer de compositions de biomasse de microalgues du genre *Chlorella* de qualité organoleptique convenable, présentant toujours la même richesse en composants d'intérêt, telles les protéines, permettant l'utilisation de celles-ci dans des produits alimentaires plus nombreux et diversifiés.

### Résumé de l'invention

La présente description est relative à un procédé de décoloration d'une biomasse de microalgues de type *Chlorella* riche en protéines, caractérisé en ce que l'on:
- produit la biomasse de microalgues en hétérotrophie et dans des conditions de fermentation continue, et
- diminue la coloration de ladite biomasse par l'augmentation du rapport des taux de croissance µ/µmax.

De préférence, l'on décolore la biomasse de microalgue en paramétrant la conduite de fermentation continue de manière à ce que le rapport des taux de croissance µ/µmax soit supérieur ou égal à 0,90, de préférence supérieur ou égal à 0,95.

De préférence, la fermentation continue est réalisée en chémostat.

De préférence, la biomasse de microalgue riche en protéines présente une teneur en protéines de plus de 50 % exprimés en N 6,25. De préférence, la microalgue de type *Chlorella* est *Chlorella protothecoides.*

### Présentation détaillée de l'invention

La présente description est donc relative à un procédé de production d'une biomasse de microalgues de type *Chlorella* riche en protéines en contrôlant la coloration de la biomasse, caractérisé en ce que l'on:
- choisit de produire la biomasse de microalgues en hétérotrophie et dans des conditions de fermentation continue,
- fait varier la coloration de ladite biomasse en contrôlant le rapport des taux de croissance µ/µmax.

Au sens de la présente invention on entend par « biomasse riche en protéines » une biomasse qui présente une teneur en protéines de plus de 50 %, de préférence de plus de 53 %, de manière encore plus préférée de plus de 55 % exprimés en N 6,25.

De préférence, les microalgues du genre Chlorella sont choisies dans le groupe constitué de *Chlorella vulgaris, Chlorella sorokiniana* et *Chlorella protothecoides,* et sont plus particulièrement *Chlorella protothecoides.* Dans un mode de réalisation particulier, la souche est *Chlorella protothecoides* (souche UTEX 250 - *The Culture Collection of Algae at the University of Texas at Austin* - USA). Dans un autre mode de réalisation particulier, la souche est *Chlorella sorokiniana* (souche UTEX 1663 - *The Culture Collection of Algae at the University of Texas at Austin* - USA).

Il a été constaté de manière tout à fait étonnante par les inventeurs que la coloration est inversement corrélée au rapport des taux de croissance µ/µmax : la coloration diminue lorsque le rapport de croissance µ/µmax augmente et elle augmente lorsque le rapport de croissance µ/µmax diminue.

La biomasse de chlorelles riches en protéines de l'invention est connue pour sa couleur verte marquée, liée à sa teneur naturelle en chlorophylle.

Au sens de l'invention, « la décoloration de la biomasse » s'entend du passage de la couleur verte de base à une couleur jaune, en passant par toutes les nuances de vert à jaune.

La mesure de la couleur verte ou jaune peut être réalisée par tout modèle colorimétrique connu en tant que tel par l'homme du métier.

Il peut être choisi le modèle TSL (acronyme de Teinte, Saturation, Luminosité) qui est basé sur le ressenti de la perception humaine, d'où son nom de modèle perceptuel.

Les trois critères qui caractérisent le TSL sont la teinte, la saturation et enfin, la luminosité. La saturation reflète bien la notion intuitive de coloration, car elle va des couleurs vives vers le gris. La luminosité est mesurée entre le noir (pas de lumière ou valeur 0) et le blanc (lumière maximum ou valeur 1).

L'avantage majeur du modèle TSL est de séparer clairement la composante luminosité des composantes chromatiques. Teintes et saturation sont sur un même plan en conformité avec la sensation colorée de l'œil et la luminosité quant à elle, est placée sur un axe perpendiculaire.

Il est également possible d'utiliser le système L,a,b établi par la Commission Internationale de l'Eclairage qui consiste en un repère cartésien tridimensionnel (L, a, b) où l'axe des « L » représente la clarté, l'axe des « a » représente une nuance de couleur entre le rouge et le vert, et l'axe des « b » représente une nuance de couleur entre le jaune et le bleu.

A titre d'illustration, les tableaux ci-dessous présentent les mesures réalisées suivant le modèle TSL et suivant le système L,a,b pour les deux couleurs vertes et jaunes à l'extrémité de la chaîne de couleur classiquement mesurées pour les biomasses selon l'invention (mesures réalisées en triple).

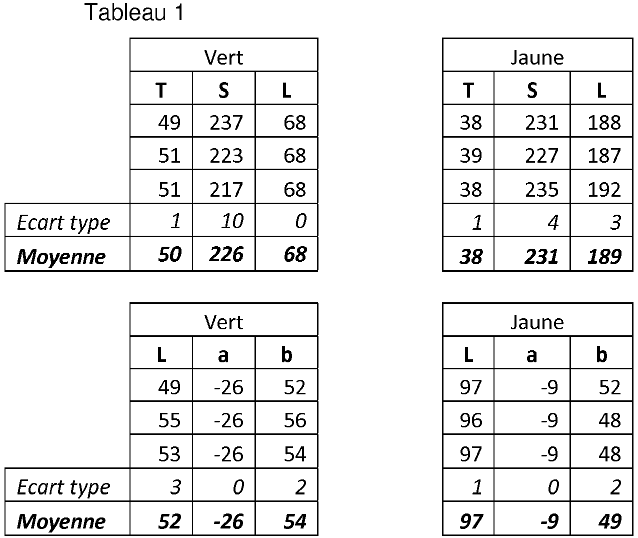

Comme il sera exemplifié ci-après, la société Demanderesse a choisi d'exprimer la couleur de la biomasse produite à l'aide du mode TSL, le modèle L, a, b étant plutôt adapté à la mesure des variations colorimétriques des poudres blanches à jaune (mesure de la balance des jaunes , axe « b » ou « Yellow index »).

Quant au procédé de fermentation continue, il faut l'entendre ici plus particulièrement comme une conduite de fermentation avec ajout du milieu stérile et soutirage réalisés à l'intérieur d'un même cycle de fermentation.

Cependant, le recours à ce procédé de fermentation continue n'est pas destiné ici à augmenter la productivité en biomasse mais, à contrôler la couleur de la biomasse produite.

La société Demanderesse a en effet trouvé que, de manière surprenante et inattendue, il était possible de faire varier la coloration de ladite biomasse par le contrôle du rapport des taux de croissance µ/µmax.

Pour illustrer ce résultat, la société Demanderesse recommande de réaliser cette fermentation continue en chémostat : le milieu frais est alors fourni à un débit constant (F), puis prélevé du fermenteur au même débit, maintenant ainsi un volume de culture constant (V).

À l'état stable, le taux de croissance « µ » de la culture est égal au taux de dilution « D », et est défini par la relation D = F/V.

La concentration de biomasse et des autres paramètres se stabilisent à des valeurs dépendant de la dynamique de la fermentation.

Fondamentalement, la culture en chémostat permet à l'homme du métier de contrôler le taux de croissance (µ) à une valeur inférieure à une valeur maximale appelée le taux de croissance maximal (µmax).

Cela contraste avec les cultures en mode batch, où la concentration de la biomasse et les conditions environnementales (en termes de pH, nutriments concentration, etc.) changent de façon significative au cours de la durée (limitée) de la fermentation, et l'homme du métier n'a aucun contrôle sur le taux de croissance.

Le facteur qui détermine le taux de croissance d'une population de cellules dans un chémostat est le taux de dilution, c'est à dire le débit d'alimentation de l'élément nutritif limitant.

Dans le procédé selon la description, il s'agit ici du glucose.

Dans un chémostat fonctionnant à un taux de dilution faible, le glucose est présent à de très faibles concentrations à l'état stationnaire.

Par conséquent, la plupart des éléments nutritifs sont transformés dans des cellules et la concentration de la biomasse est élevée (proche de la concentration cellulaire d'une culture batch équivalent à la fin de la phase exponentielle).

Lorsque le taux de dilution augmente, la disponibilité du glucose augmente; toutefois, la vitesse d'élimination des cellules du fermenteur est également plus élevée et ainsi la concentration de la biomasse chute.

A des valeurs de D proche de µmax, la limitation en nutriments disparaît entièrement car il y a trop peu de cellules dans la culture qui utilisent les nutriments disponibles.

Les cellules restantes dans le fermenteur croissent à leur taux de croissance maximum puisque même le nutriment limitant est présente en excès.

Enfin, à D supérieur à µmax, un état d'équilibre ne peut plus être maintenu et le nombre de cellules dans les fermenteurs commence à baisser puisque la vitesse à laquelle de nouvelles cellules sont produites est insuffisante pour éviter la dilution par l'addition de milieu frais, ce qui conduit au lavage des cellules du fermenteur.

Ces données permettent d'expliquer la raison pour laquelle si la fermentation en chémostat est préconisée pour augmenter la productivité à glucose résiduel nul, l'homme du métier choisira de conduire le chémostat de manière à ce que D soit inférieure au µmax afin d'obtenir la concentration la plus élevée en biomasse.

Pour sa part, la société Demanderesse a trouvé que plus on augmentait µ vers la valeur du µmax, plus on augmentait la productivité mais également plus on décolorait la biomasse produite.

Comme il sera exemplifié ci-après, les paramètres utilisés dans cette fermentation en continu sont, en fonction des conditions exemplifiées ci-après, les suivants :
- pH = 5.2 régulé à l'ammoniaque 10 % (v/v),
- pO₂ = 30 % régulé en cascade sur l'agitation,
- aération : 1 vvm (1,5 l/min).

A l'équilibre :
- débit d'alimentation = débit de sortie = 0,120 l/h à 0,180 l/h
- taux de dilution = µ =0,02 h⁻¹ à 0,12 h⁻¹
- concentration en biomasse = 50 g/l à 100 g/l
- concentration en glucose = 100 g/l à 200 g/l

Dans un exemple, le chémostat est équilibré durant 5 renouvellements (62,5 h si D = 0,08 h⁻¹). Des prélèvements sont réalisés au terme des 5 renouvellements et plus de 7 h ensuite, afin de vérifier l'état d'équilibre du chémostat.

Il est ainsi trouvé que la biomasse de *Chlorella protothecoides* de base, de couleur verte, va être progressivement décolorée vers la couleur jaune si on augmente le µ de 0,08 h⁻¹ à 0,1 h⁻¹.

A l'inverse, si on réduit le µ de 0,08 h⁻¹ à 0,06 h⁻¹ la coloration de la biomasse est renforcée vers les tons verts.

Par ailleurs, et c'est ce qui est primordial dans le procédé conforme à la description, cette conduite de fermentation en chémostat n'altère pas la composition de la biomasse produite.

Comme il sera exemplifié ci-après, que le taux de croissance soit faible (0,06 h⁻¹) ou fort (0,1 h⁻¹), il n'a pas été constaté d'effet important sur la composition de la biomasse de *Chlorella protothecoides* produite.

Les principales différences sont plutôt au niveau des vitesses de production qui sont globalement améliorées avec un µ fort.

A titre d'exemple, pour *Chlorella protothecoides,* les meilleurs résultats obtenus permettent d'atteindre une productivité de plus de 9,5 g de biomasse/l/h en préservant une teneur en protéines exprimées en % de N6,25 de plus de 55 %.

Une fois encore, il est plus étonnant de constater que la variation du µ a un impact important sur la couleur de la biomasse produite.

Sans être liée par une quelconque théorie, la société Demanderesse considère, en regard des résultats obtenus (sur la base de la représentation par le modèle TSL de la teinte T de la biomasse en fonction de la luminosité L pour chaque mesure), que :
- les variations de la teinte pourraient correspondre à la diminution progressive de la production de chlorophylle qui masque les autres pigments (caroténoïdes),
- la luminosité, quant à elle, pourrait davantage correspondre à la diminution de la concentration globale en pigments.

Comme il sera exemplifié ci-après, le niveau de couleur considéré « correct » est celui pour lequel (pour une gamme de teinte comprise entre 30 et 60 et une gamme de luminosité comprise entre 50 et 230) :
- la valeur de teinte est inférieure à 40,
- la valeur de luminosité est supérieure à 135, de préférence supérieure à 150 et de manière encore plus préférée supérieure ou égale à 170.

L'invention sera mieux comprise à l'aide des exemples.

### Description des figures

Figure 1 : Evolution de l'O₂ consommé et du CO₂ produit en fonction du taux de dilution imposé.
Figure 2 : Evolution de l'absorbance, du glucose et de la teneur en biomasse en fonction du taux de dilution imposé.
Figure 3 : Evolution de la teinte et de la luminosité en fonction du taux de dilution imposé.
Figure 4 : Evolution de la teinte en fonction du rapport des taux de croissance µ/µmax.
Figure 5 : Evolution de la luminosité en fonction du rapport des taux de croissance µ/µmax.

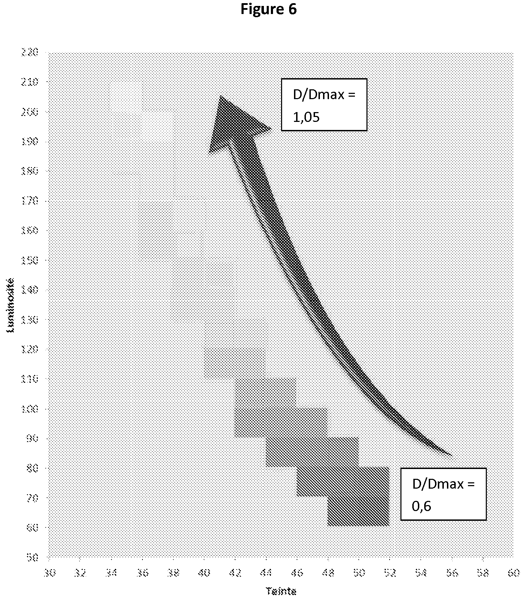

Figure 6 : Evolution de la teinte et de la luminosité en fonction du rapport des taux de dilution D/Dmax.

### Exemples

### Exemple 1 : Détermination de la valeur de umax et évolution de la couleur de la biomasse en fonction du ratio u/umax en accélérostat

La souche utilisée est *Chlorella protothecoides UTEX250 (The Culture Collection of Algae at the University of Texas at Austin* - USA).

La fermentation est réalisée en accelerostat, variante du chémostat où le fermenteur n'entre jamais en équilibre dynamique stationnaire. En fait, le D est augmenté de manière linéaire et progressive en partant d'une valeur basse.

L'intérêt de cette technique est l'étude d'une large gamme de taux de dilution ainsi que l'accès rapide et précis au µmax de la souche dans les conditions mises en œuvre.

Les conditions de fermentation sont les suivantes, pour la production de 100 g/l de biomasse :

### Milieu d'alimentation

- Glucose : 200 g/l
- (NH₄)₂SO₄: 1 g/l
- NH₄H₂PO₄ : 8 g/l
- MgSO₄, 7 H₂O : 4,8 g/l
- FeSO₄, 7 H₂O : 0,020 g/l
- CaCl₂, 7 H₂O : 0,050 g/l
- ZnSO₄, 7 H₂O : 0,025 g/l
- MnSO₄, 1 H₂O : 0,020 g/l
- CuSO₄, 5 H₂O : 0,001 g/l
- Thiamine.HCl: 0.020 g/l
- Biotine : 0.001 g/l
- Pyridoxine : 0.010 g/L

### Conduite de fermentation :

- Mise à pH 5,2 avec NH₄OH 10 % (p/v)
- Température d'incubation : 28°C
- Inoculum : 0,5 l d'un Erlen de préculture présentant une DO_{750 nm} = 15 dans un fermenteur de 2 l (1,5 l de milieu hors agitation avant ajout)
- Pulse d'antimousse SIGMA S208 50% éthanol (v/v) au rythme d'un pulse d'1 seconde par heure
- Agitation : 300 rpm au départ
- Aération : 1,5 l/min d'air
- Régulation pO₂: 30 %

L'analyse des gaz (O₂/CO₂), l'absorbance à 750 nm, la concentration en glucose et le poids de cellules sont les paramètres mesurés afin de déterminer la valeur du µmax.

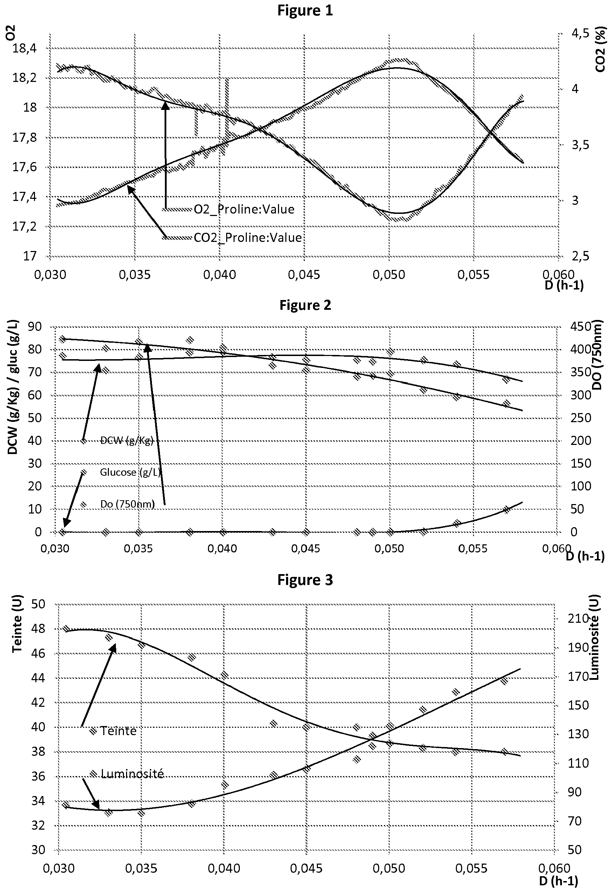

La figure 1 présente l'évolution de l'O₂ consommé et du CO₂ produit en fonction du taux de dilution imposé.

Il apparait que l'O₂ et le CO₂ évoluent progressivement jusque 0,05 h-1, puis les courbes s'inversent brusquement.

En parallèle des mesures de gaz, l'évolution de l'absorbance, du glucose et de la teneur en biomasse sont suivis de manière cinétique.

La figure 2 présente les résultats obtenus.

Une fois encore, à partir de 0,05 h-1 il y a :
- début d'accumulation de glucose résiduel,
- infléchissement de la courbe de biomasse (initialement stable à 75 g/l de cellules sèches),

ce qui indique que le chémostat commence à entrer en phase de lessivage, c'est-à-dire que le µ imposé devient plus élevé que le µmax de la souche dans les conditions imposées.

Le système élimine donc plus vite les cellules qu'elles ne se reproduisent, ce qui provoque la diminution de la charge en cellules du réacteur, la remontée de l'O₂ non consommé et la diminution du CO₂ produit.

La baisse de l'absorbance traduit également la baisse de la teneur en biomasse à partir d'un µ de 0,05 h⁻¹.

Il résulte de toutes ces analyses que le Dmax = µmax = 0,05 h⁻¹, dans les conditions de fermentation choisie.

Les mesures de couleur de la biomasse produite sont effectuées selon le modèle TSL.

La figure 3 présente l'évolution de la couleur (teinte et luminosité) en fonction des taux de dilution.

L'évolution des courbes de teinte et de luminosité est directement dépendante du µmax.

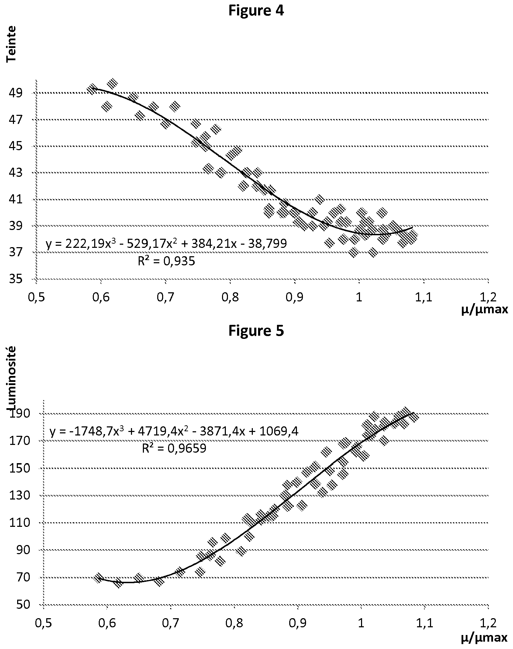

Trois représentations graphiques permettent d'illustrer l'évolution de la couleur en fonction du rapport µ/µmax :
- évolution de la teinte en fonction du rapport µ/µmax (figure 4),
- évolution de la luminosité en fonction du rapport µ/µmax (figure 5),

Il s'en déduit que :
- La couleur évolue très fortement tout au long de l'accelerostat (c'est-à-dire en fonction de la valeur du D),
- La biomasse la plus jaune et décolorée est obtenue lorsque le µ tend vers le µmax, dans les conditions opératoires choisies.

### Exemple 2 : Evolution de la couleur de la biomasse en fonction du taux de dilution (et donc de la valeur de u) de points d'équilibre en chémostat

Des chémostats sont réalisés dans les conditions identiques à l'exemple 1 mais avec un milieu deux fois moins concentré de sorte que la concentration en biomasse à l'équilibre est de 50 g/l. Le µ max sur ce milieu est 0.104 h⁻¹.

Les mesures suivantes ont été réalisées à différents taux de dilution une fois l'équilibre obtenu (5 renouvellements). Elles confirment l'effet du ratio µ/µmax sur la coloration :

**Tableau 2**

| Essais | 1 | 2 | 3 | 4 |
|---|---|---|---|---|
| D (h-1) | 0.102 | 0.028 | 0.088 | 0.064 |
| µ/µmax | 0.98 | 0.27 | 0.85 | 0.61 |
| Teinte | 36 | 52 | 47 | 48 |
| Luminosité | 171 | 68 | 86 | 81 |

Inversement, la composition de la biomasse n'est pas significativement modifiée comme le montre les teneurs en protéines (N 6.25 et Acides Aminés Totaux), acides gras et sucres totaux qui sont donnés ci-dessous.

**Tableau 3**

| Essais | 1 | 2 | 3 | 4 |
|---|---|---|---|---|
| N 6.25 (%sec) | 60,0 | 56,1 | 54,2 | 57,2 |
| Acides Aminés totaux (%sec) | 37,6 | 40,6 | 39,5 | 40,6 |
| Acides Gras totaux (%sec) | 8,4 | 7,0 | 7,2 | 7,6 |
| Sucres totaux (%sec) | 31,2 | 24,8 | 30,1 | 26,2 |

## Revendications

1. Procédé de décoloration d'une biomasse de microalgues de type *Chlorella* riche en protéines, **caractérisé en ce que** l'on produit la biomasse de microalgues en hétérotrophie et dans des conditions de fermentation continue, en diminuant la coloration de ladite biomasse par l'augmentation du rapport des taux de croissance µ/µmax, de sorte que la valeur de teinte est inférieure à 40 et la valeur de luminosité est supérieure à 135, la teinte (T) et la luminosité (L) étant définies dans le modèle perceptuel colorimétrique TSL.

2. Procédé selon la revendication 1, **caractérisé en ce que** l'on décolore la biomasse de microalgues en paramétrant la conduite de fermentation continue de manière à ce que le rapport des taux de croissance µ/µmax soit supérieur ou égal à 0,90.

3. Procédé selon la revendication 1, **caractérisé en ce que** l'on décolore la biomasse de microalgues en paramétrant la conduite de fermentation continue de manière à ce que le rapport des taux de croissance µ/µmax soit supérieur ou égal à 0,95.

4. Procédé selon l'une quelconque des revendications 1 à 3, **caractérisé en ce que** l'élément nutritif limitant du milieu d'alimentation de la biomasse de microalgues est le glucose.

5. Procédé selon l'une quelconque des revendications 1 à 4, **caractérisé en ce que** la fermentation continue est réalisée en chémostat.

6. Procédé selon l'une quelconque des revendications 1 à 5, **caractérisé en ce que** la biomasse de microalgues riche en protéines présente une teneur en protéines de plus de 50 % exprimés en N 6,25.

7. Procédé selon l'une quelconque des revendications 1 à 6, **caractérisé en ce que** la microalgue de type *Chlorella* est *Chlorella protothecoides.*

## Patentansprüche

1. Verfahren zum Bleichen einer proteinreichen Biomasse von Mikroalgen der Gattung *Chlorella,* **dadurch gekennzeichnet, dass** die Mikroalgenbiomasse heterotroph und unter Bedingungen einer kontinuierlichen Fermentation produziert ist und die Färbung besagter Biomasse durch die Erhöhung des Verhältnisses der Wachstumsraten µ/µmax verringert ist, so dass der Farbtonwert kleiner 40 und der Helligkeitswert größer 135 ist, wobei der Farbton (T) und die Helligkeit (L) im Farbwahmehmungsmodell TSL definiert sind.

2. Verfahren gemäß Anspruch 1, **dadurch gekennzeichnet, dass** man die Mikroalgenbiomasse bleicht, indem die Steuerung der kontinuierlichen Fermentation derart eingestellt ist, so dass das Verhältnis der Wachstumsraten µ/µmax höher oder gleich 0,90 ist.

3. Verfahren gemäß Anspruch 1, **dadurch gekennzeichnet, dass** man die Mikroalgenbiomasse bleicht, indem die Steuerung der kontinuierlichen Fermentation derart eingestellt ist, so dass das Verhältnis der Wachstumsraten µ/µmax höher oder gleich 0,95 ist.

4. Verfahren gemäß einem der Ansprüche 1 bis 3, **dadurch gekennzeichnet, dass** die limitierende Nährstoffkomponente des Nährmediums der Mikroalgenbiomasse die Glucose ist.

5. Verfahren gemäß einem der Ansprüche 1 bis 4, **dadurch gekennzeichnet, dass** die kontinuierliche Fermentation in einem Chemostaten durchgeführt wird.

6. Verfahren gemäß einem der Ansprüche 1 bis 5, **dadurch gekennzeichnet, dass** die proteinreiche Mikroalgenbiomasse einen Proteingehalt von mehr als 50%, ausgedrückt als N 6,25 aufweist.

7. Verfahren gemäß einem der Ansprüche 1 bis 6, **dadurch gekennzeichnet, dass** die Mikroalge der Gattung *Chlorella Chlorella protothecoides* ist.

## Claims

1. A process for bleaching a biomass of *Chlorella* microalgae rich in proteins, **characterized in that** the microalgal biomass is produced under heterotrophic conditions and under continuous fermentation conditions, while decreasing the coloring of said biomass by increasing the µ/µmax growth rate ratio, so as the hue value is less than 40 and the lightness value is greater than 135, the hue (H) and the lightness (L) being defined by the colorimetric perceptual model (HSL).

2. The process as claimed in claim 1, **characterized in that** the microalgal biomass is bleached by parameterizing the conducting of continuous fermentation in such a way that the µ/µmax growth rate ratio is greater than or equal to 0.90.

3. The process as claimed in claim 1, **characterized in that** the microalgal biomass is bleached by parameterizing the conducting of continuous fermentation in such a way that the µ/µmax growth rate ratio is greater than or equal to 0.95.

4. The process as claimed in any one of claims 1 to 3, **characterized in that** the limitating nutritive element of the feed medium of the microalgal biomass is glucose.

5. The process as claimed in any one of claims 1 to 4, **characterized in that** the continuous fermentation is carried out in a chemostat.

6. The process as claimed in any one of claims 1 to 5, **characterized in that** the microalgal biomass rich in proteins has a protein content of more than 50%, expressed in N 6.25.

7. The process as claimed in any one of claims 1 to 6, **characterized in that** the *Chlorella* microalga is *Chlorella protothecoides.*
